# EUROPEAN PATENT APPLICATION

(11) **EP 4 759 252 A1**
(43) Date of publication of application: **17.06.2026**
(21) Application number: 24953275.5
(22) Date of filing: 05.11.2024
(51) Int. Cl.: A61B 17/88, A61B 17/70, A61B 17/16, A61B 17/17, A61B 34/20, A61B 17/00

(54) **SURGICAL TOOL ADAPTER, SURGICAL DRIVER TO WHICH SAME IS APPLIED, AND METHOD FOR MOUNTING TOOL ON DRIVER**

(30) Priority: 05.09.2024 KR 20240121132; 21.10.2024 KR 20240144323
(71) Applicant: Curexo, Inc., Seoul 05814 (KR)
(72) Inventor: CHA, Yong Yeob, Seoul 05814 (KR)
(74) Representative: Lavoix
(86) International application number: PCT/KR2024/017268
(87) International publication number: WO 2026/054167

(57) **Abstract**

A surgical tool adapter, a surgical driver including the same, and a method of mounting a tool on the surgical driver are disclosed, wherein the surgical tool adapter may include a rotational body having a socket portion to which a surgical tool with a shaft is detachably coupled, a stationary body having a bore cavity in which the rotational body is rotatably installed, and a shaft aligner configured to control radial runout of the tool shaft by aligning the shaft coaxially with a longitudinal axis of the rotational body while allowing rotation of the rotational body and the coupled shaft in the bore cavity of the stationary body.

## Description

### Technical Field

The present disclosure relates to a surgical tool adapter, and more particularly, to a tool adapter compatible with surgical tools having shafts of different sizes, a tool driver including the tool adapter, and a method of mounting a tool on the tool driver.

### Background Art

Various rotary tools such as screwdrivers, tapping tools, and reamers may be used in surgical operations. In surgical operations, for example, spinal fusion surgeries, manual surgical drivers are used to fix a screw at a surgical site, perform tapping, i.e., female screw machining, or simply form a hole by using rotary tools such as screw tips, drill tips, or tap bits provided at a shaft tip for specific tasks or machining purposes.

Some of these manual surgical drivers are provided with navigation markers for surgical navigation, which are useful for providing real-time position and orientation information about the surgical tool to a surgeon.

The stems of tools used in such surgical drivers, i.e., tool shafts or tool shanks, have different specifications depending on the manufacturer, and therefore, surgical drivers must be prepared for each shank specification. Such tool shafts having different specifications each ultimately require individual surgical drivers, and therefore cause a drawback in terms of cost and a spatial drawback of increasing the number of surgical tools in an operating room.

### Disclosure of the Invention

### Technical Problem

The present disclosure provides a surgical tool adapter compatible with surgical tools of different specifications, a surgical tool driver including the same, and a method of mounting surgical tools on the surgical tool driver.

The present disclosure provides a surgical tool adapter configured to securely fix shafts of surgical tools having different diameters while allowing rotation of the shafts without radial runout, a surgical tool driver including the same, and a method of mounting tools on the surgical tool driver.

### Solution to Problem

An adapter for compatably mounting surgical tools according to the present disclosure includes:
a rotational body having a socket portion into which a coupling head provided on a tool shaft is detachably coupled;
a stationary body having a bore cavity in which the rotational body is rotatably mounted; and
a shaft aligner configured to align the tool shaft coaxially with a longitudinal axis of the rotational body while allowing rotation of the rotational body and the coupled shaft within the bore cavity of the stationary body, thereby controlling radial runout of the tool shaft.

According to one or more embodiments, the shaft aligner may include
a plurality of actuators are movably disposed to be contactable with and detachable from a rotational support surface defined on an outer circumferential surface of the tool shaft to support rotation of the shaft; and
a positioner configured to align the plurality of movable actuators with the rotational support surface of the tool shaft.

According to one or more embodiments, the plurality of actuators may be arranged around the shaft such that they are movable in a radial direction with respect to a longitudinal central axis of the tool shaft to approach and move away from the rotational support surface of the tool shaft.

According to one or more embodiments, the plurality of actuators may be rotary members that perform sliding or rolling movement on the rotational support surface of the tool shaft.

According to one or more embodiments, the plurality of actuators may have a shape of one of a ball, a cylinder, a cone, or a truncated cone having a surface partially contacting the rotational support surface.

According to one or more embodiments, around an opening of the socket portion into which the tool shaft is inserted a plurality of actuation holes into which the plurality of actuators are radially movable may be formed at arbitrary angular intervals,
the positioner may include a cam member configured to press or release the plurality of actuators located in the plurality of actuation holes so that each of the plurality of actuators comes into contact with or is separated from a rotational support surface of the tool shaft in the socket portion, and
the cam member may have an inclined cam surface configured to push the plurality of actuators toward or away from a center of the tool shaft.

According to one or more embodiments, the cam member may be provided to be movable in a longitudinal direction of the rotational body and configured such that depending on a position of the cam member in the longitudinal direction the plurality of actuators are pushed toward or moved away from a center of the tool shaft.

According to one or more embodiments, the surgical tool adapter may further include a locking tube, which is integrally coupled with the stationary body and forms a portion of a bore cavity accommodating the rotational body, and

The cam member may be coupled to one end of the rotational body to be movable in the longitudinal direction thus may be configured for the plurality of actuators to be brought into contact with or to be separated from the rotational support surface depending on a relative position in the longitudinal direction with respect to the rotational body.

According to one or more embodiments, an actuation hole in which a locking actuator for restraining a portion of the coupling head inserted into the socket portion is positioned may be formed in the rotational body,
a cam surface configured to constrain or release the locking actuator may be formed on one inner side of the locking tube corresponding to the actuation hole,

Thus, depending on a position of the rotational body in the longitudinal direction with respect to the rotational body, the locking actuator may be constrained by or released from the cam surface, thereby enabling one side of the coupling head of the tool shaft coupled to the socket portion to be fixed or released from the socket portion.

According to one or more embodiments, the stationary body, the cam member and the locking tube therebetween may be disposed substantially concentrically on a single axis,
the bore cavity may accommodate the rotational body to be reciprocally movable along the longitudinal axis of the the bore cavity, thus may be configured for the plurality of actuators interfering with the rotational body to be disengaged from the cam surface as the rotational body moves in the longitudinal direction thereby releasing locking of the tool shaft in the socket portion,
a compression spring elastically biasing the rotational body may be positioned within the locking tube to provide a reaction force against movement of the rotational body in the longitudinal direction thereby restoring a position of the rotational body, and
the cam member may be threadably coupled to one longitudinal end of the locking tube thus may be configured to move a position of the cam surface with respect to the plurality of actuators in the longitudinal direction according to a rotational position of the cam member with respect to the locking tube.

According to one or more embodiments, the positioner may include
a cam member having a cam surface surrounding an array of the plurality of actuators, and
a cam holder configured to fix a position of the cam member as determined by the positioner,
wherein the cam surface may be inclined with respect to a longitudinal axis of the tool shaft to press or release the plurality of actuators with respect to the rotational support surface of the tool shaft.

According to one or more embodiments, the positioner may include
a linear transfer unit configured to be reciprocally movable along the rotational axis with respect to the stationary body and a cam holder including a set screw to fix the cam member.

According to one or more embodiments, a socket portion may be located on the longitudinal axis of the rotational body, and
a plunger configured to press a top surface of the coupling head may be provided on a bottom of the socket portion corresponding to the top surface of the coupling head.

According to one or more embodiments, The plunger may be elastically biased by a spring and may be configured to elastically press the top surface of the coupling head.

In a surgical tool driver according to the present disclosure
a hollow hole, a centering dimple, a centering pocket, or a centering recess into which a portion of the plunger enters may be formed on the top surface of the coupling head.

A surgical tool driver according to one or more embodiments may include
a surgical tool having a shaft provided with a tool tip at a front end and a coupling head at a rear end,
a surgical tool adapter including a rotational body to which the surgical tool is detachably coupled, and
a handle coaxially connected to a rotational rod provided in the rotational body.

In a surgical tool driver according to one or more embodiments,
a shank may be coaxially coupled to the rotational body, and a handle having a ratchet capable of controlling a rotational direction may be detachably coupled to the shank.

In a surgical tool driver according to one or more embodiments,
a navigation marker for surgical navigation may be attached to the stationary body.

In a surgical tool driver according to one or more embodiments,
the navigation marker may include
a plurality of optically detectable markers,
a marker plate to which the plurality of markers are fixed, and
a support post connecting the marker plate to the stationary body.

According to the present disclosure, a method of compatibly mounting surgical tools to a surgical tool driver may include
preparing a surgical tool having a tool shaft provided with a tool tip at a front end and a coupling head at a rear end,
installing a rotational body rotatably within a bore cavity formed in a stationary body,
coupling the coupling head of the shaft to a socket portion provided in the rotational body, and
aligning the tool shaft with the rotational body by controlling radial runout of the tool shaft using a driver shaft aligner, while supporting the tool shaft coupled to the rotational body to be rotatable with respect to the stationary body and allowing rotation of the rotational body and the coupled tool shaft in the bore cavity of the stationary body.

In a method according to one or more embodiments, a cam member having a cam surface surrounding an array of the plurality of actuators may be operated, thus the plurality of actuators may rotatably support the rotational support surface of the tool shaft using the cam surface.

In a method according to one or more embodiments, the cam member may be moved in one or another direction along a longitudinal axis of the tool shaft, thus the actuator may be pressed against or released from the rotational support surface of the tool shaft using the cam surface formed obliquely with respect to the longitudinal axis of the tool shaft.

### Description of Drawings

The accompanying drawings and photographs illustrate an overall or partial view of the surgical driver according to the present disclosure or specific components thereof, and the embodiments illustrated in these drawings do not limit the technical scope of the present disclosure.
FIG. 1 illustrates a surgical tool driver according to the present disclosure.
FIG. 2 illustrates various types of surgical tools.
FIG. 3 illustrates screws used in surgical operations such as spinal fusion surgery.
FIG. 4 illustrates an adapter equipped with a navigation marker unit according to an embodiment of the present disclosure.
FIG. 5(A) is a partial front view of a head portion of a tool shaft, and FIG. 5 (B) is a sectional view thereof.
FIG. 6 is a schematic exploded perspective view of an adapter according to an embodiment of the present disclosure.
FIG. 7 is a schematic sectional view of the adapter of FIG. 6 in an assembled state.
FIG. 8A is a partial view showing a state in which a tool shaft is rotatably supported in the adapter illustrated in FIG. 5.
FIG. 8B is a partially enlarged view showing a state in which alignment balls support the support surface of the tool shaft in a rollable or slidable manner, and
FIG. 9 is a schematic sectional view of an adapter according to an embodiment of the present disclosure, showing a state in which a tool shaft is inserted or removed.

### - Description of Reference Numerals -

100: Surgical driver
110: Adapter
111: Stationary body
111bc: Bore cavity
112: Holding button
112sp: Bias spring
113: Cover
114: Rotational body
114sh: Shank
115: Locking tube
115th: Threaded portion of the locking tube
116: Centering plunger
116sp: Bias spring
117sp: Bias spring
118: Shaft aligner
118cn: Cam member or cam nut
118th: Threaded portion
118ec: End cap
118ab: Actuator or alignment ball
120: Navigation marker unit
123: Navigation marker
124: Navigation marker fixing plate
125: Navigation marker fixing post
126: Navigation marker fixing bolt
130: Surgical tool
131: Tip or bit
132: Coupling head
133: Tool shaft
133rg: Rotational support surface

### Mode for the Invention

Hereinafter, preferred embodiments of the present inventive concept are described in detail with reference to the accompanying drawings. However, the embodiments of the present inventive concept may be modified into various other forms, and the scope of the present inventive concept shall not be construed as being limited to the embodiments described below in detail. It is desirable that the embodiments of the present inventive concept be interpreted as being provided to more fully explain the present inventive concept to one of ordinary skill in the art. The same signs denote the same elements throughout. Furthermore, various elements and areas in the drawings are schematically drawn. Therefore, the present inventive concept is not limited by the relative sizes or distances illustrated in the accompanying drawings.

Terms such as first, second, etc. may be used to describe various components, but the components are not defined by these terms. The terms are used only for distinguishing one element from another element. For example, without deviating from the scope of the claims of the present inventive concept, a first element may be referred to as a second element, and vice versa.

The terms used in the present specification are used only for describing particular embodiments and are not intended to limit the present inventive concept. A singular expression may include a plural expression, unless an apparently different meaning is indicated in the context. With respect to the present application, it will be further understood that the expressions "comprises" and "comprising" used herein specify the presence of stated features, integers, steps, operations, members, components, and/or groups thereof, but do not preclude the presence or addition of one or more other features, integers, operations, members, components, and/or groups thereof.

Unless otherwise defined, all of the terms used herein including technical terms and scientific terms have the same meaning as the meaning commonly understood by one of ordinary skill in the art. Also, the terms commonly used and having the meanings as defined in dictionaries shall be understood to have consistent meanings with the corresponding terms in the context of relevant arts, and unless explicitly defined so herein, the meanings of the terms shall not be understood to be excessively formal.

In cases where an embodiment may be implemented differently, the specific process steps may be performed in an order different from the described sequence. For example, two consecutively described steps may be performed substantially simultaneously or in a reverse order to the described sequence.

Hereinafter, a surgical tool adapter according to one or more embodiments, a tool driver to which the adapter is applied, and a method of mounting a tool on the adapter will be described.

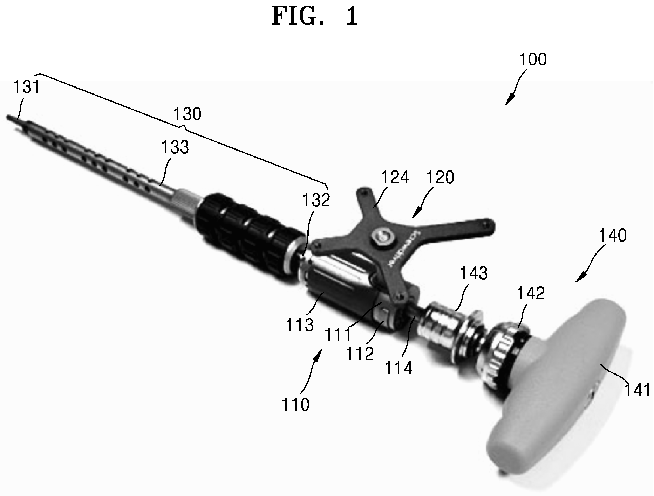

FIG. 1 illustrates a surgical tool driver according to the present disclosure.

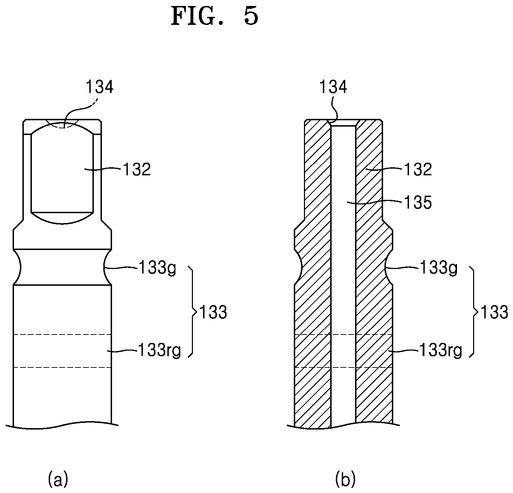

Referring to FIG. 1, a surgical tool driver 100 may include an adapter 110 compatible with different types of tools. A stationary body 111 of the adapter 110 may be equipped with a navigation marker unit 120. A surgical tool 130 and a rotary handle 140 may be coaxially connected to opposite sides of the adapter 110. More specifically, a bore cavity may be formed within the stationary body 111 of the adapter 110, and a rotational body 114 (in FIG. 5), which will be described later, may be installed therein so as to be rotatable about a longitudinal axis and reciprocally movable along the longitudinal axis. This will be described in more detail below.

The stationary body 111 may serve as a portion of the surgical tool driver 100 held by a surgeon with one hand, and the rotary handle 140 may serve as a grip portion held with the other hand by the surgeon to rotate the surgical tool 130.

The navigation marker unit 120, which is an optional component, includes a plurality of marker balls 123 and a marker fixing plate 124 on which the marker balls 123 are secured, and serves as a component of an optical navigation device for tracking and monitoring a position of the surgical tool 130 during a surgical operation.

The rotary handle 140 may include a grip 141 to be rotated by surgeon's hand, a rotation direction control ratchet 142 for transmitting the rotation force of the grip in a single direction, and a coupler 143 detachably coupled to a shank 114sh of the rotational body 114 of the adapter 110.

The surgical tool driver 100 having such an example structure may include the adapter 110 according to the present disclosure, which is compatible with tools having different specifications, particularly different shaft diameters or coupling head sizes at rear ends of shafts.

That is, the adapter according to the present disclosure includes:
the rotational body 114 with a socket portion to which a coupling head provided on a tool shaft is detachably coupled;
the stationary body 111 with a bore cavity 111bc in which the rotational body 114 may be rotatably installed; and
a shaft aligner 118 configured to coaxially align the tool shaft 133 with the longitudinal axis of the rotational body 114 while allowing rotation of the rotational body 114 and the tool shaft 133 coupled thereto within the bore cavity 111bc of the stationary body 111, to control radial runout of the tool shaft.

In the above configuration, in compatibly mounting tool shafts of different specifications to the rotational body, the shaft aligner performs concentric alignment, within a certain range, of a rotational center axis of a shaft having a different specification with a rotational center axis of the rotational body rotating relative to the stationary body. The components may be implemented in various embodiments.

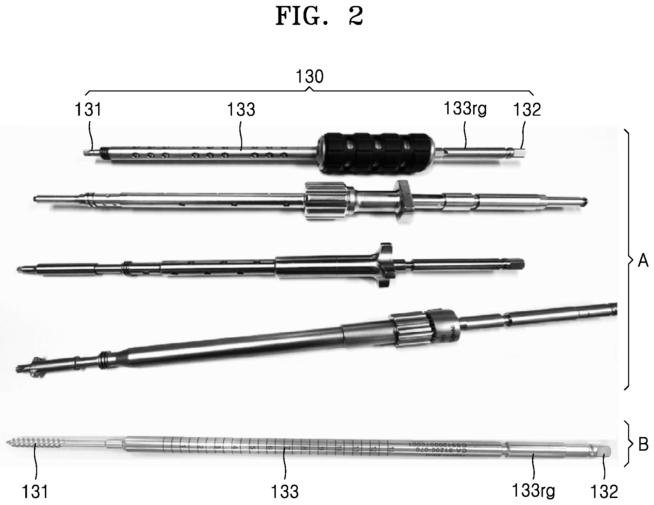

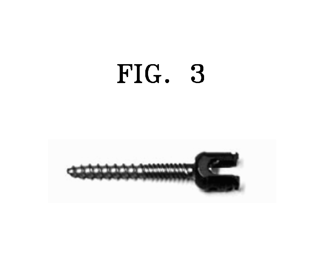

FIG. 2 illustrates various types of surgical tools, and FIG. 3 illustrates a screw used in surgical operations such as spinal fusion surgery.

The adapter 110 is compatible with various types of tools as illustrated in FIG. 2. The tools illustrated in FIG. 2 commonly include a tool shaft 133 at a middle portion, a tip or bit 131 at a front end (left end in the figure), and a coupling head 132 at a rear end (right end in the figure). In FIG. 2, reference numeral 133rg denotes a rotational support surface supported by alignment balls acting as movable actuators in the socket portion of the adapter 110 when the surgical tool 130 rotates.

Coupling heads formed or provided at rear ends of surgical tools are available in various types including 1/4-inch square chucks or heads, Hudson heads, and Jacobs chucks.

In an embodiment of the present disclosure, a square head, for example, a 1/4-inch square head, is described as a basis, but it is clear that the technical scope of the present disclosure is not limited by any specific form. The adapter 110 according to the present disclosure may support the tool shaft 133 so as to be rotatable without radial axis shake relative to the longitudinal axis, that is, the rotational center axis of the rotational body 114, even when the tool shaft has a diameter difference or tolerance of about 1 mm, by using the adapter 110 equipped with the shaft aligner.

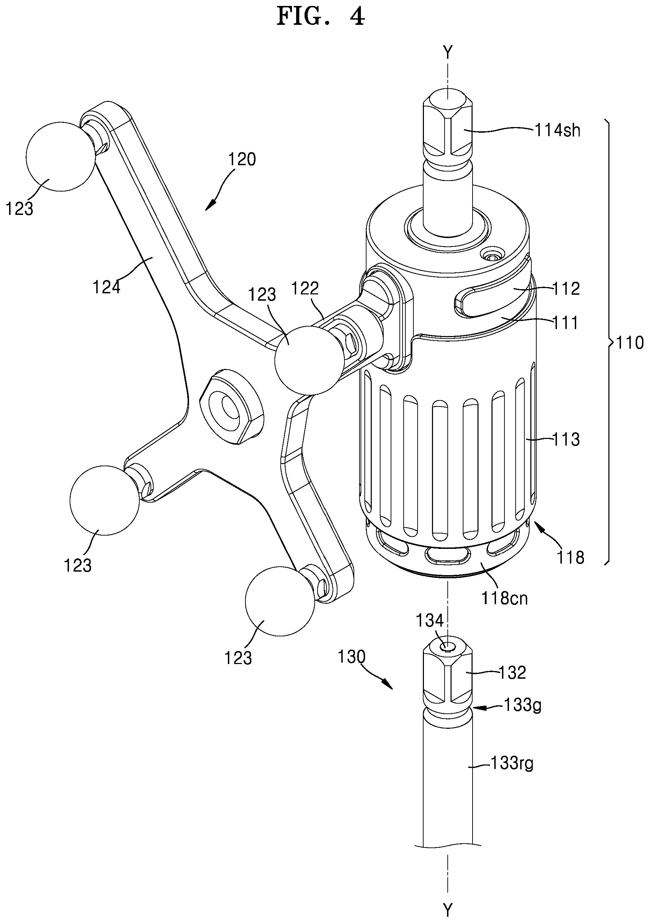

FIG. 4 illustrates the adapter 110 equipped with the navigation marker unit 120 according to an embodiment of the present disclosure.

The adapter 110 illustrated in FIG. 4 has the structure in which the navigation marker unit 120 as described above is attached. However, the navigation marker unit 120 is an optional component, and may be rigidly fixed to or detachably coupled to the body of the adapter 110, that is, the stationary body 111.

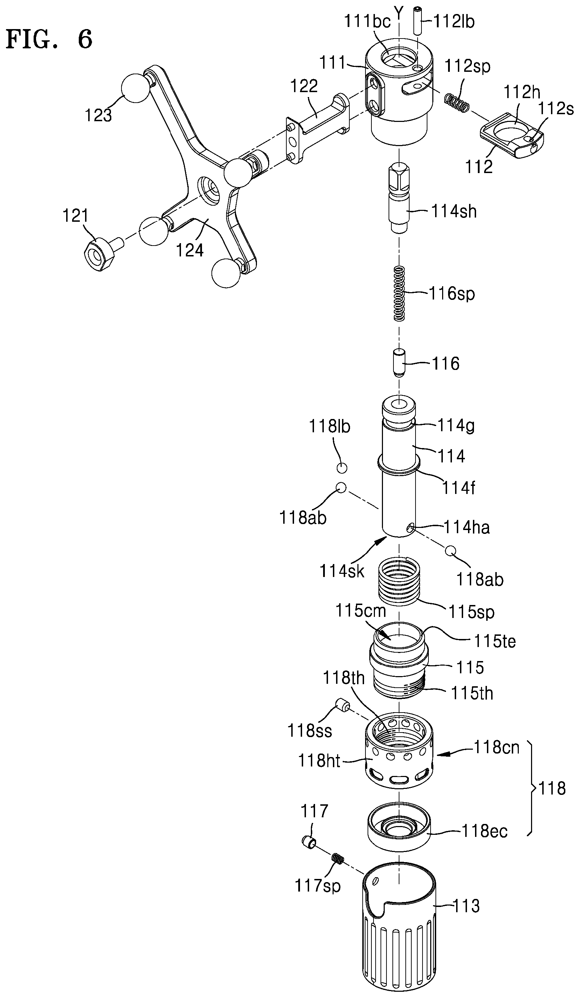

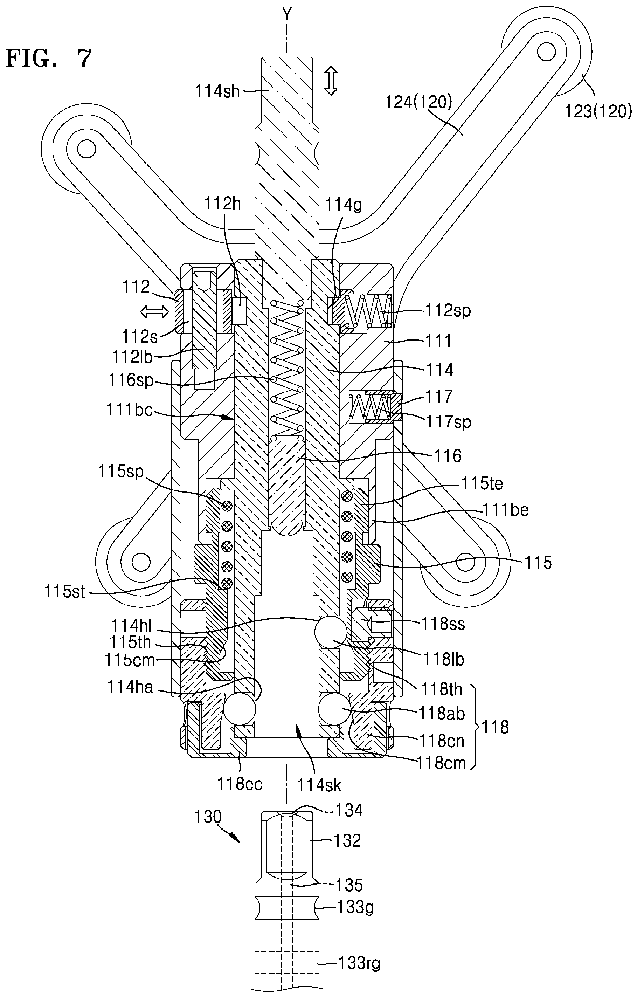

First, referring to the adapter 110, one side (lower side in the drawing) of the stationary body 111 may be protected by a cover 113. The cover 113 serves to protect the shaft aligner 118 that includes a cam nut 118cn, alignment balls 118ab, and other associated components in the vicinity, and this will be more clearly understood from the descriptions of FIGS. 6 and 7.

As described above, the shaft aligner 118 may support the tool shaft 133 so as to be rotatable without radial axis shake relative to the longitudinal axis(Y-Y) as the rotational center axis of the rotational body 114, and this will be described in further detail later.

In an upper portion of the tool shaft 133, the coupling head 132 is provided, with a centering dimple 134 as an optional element formed on the top surface thereof, and below the coupling head 132, a locking groove 133g of annular shape is formed, in which a locking ball 118lb (FIG. 6), described later, is positioned, and below the locking groove 133g, the rotational support surface 133rg is defined. At a bottom of the centering dimple 134, a hollow hole 135 passing in a longitudinal direction through the tool shaft 133 is formed, and thus, the centering dimple 134 is formed to surround the periphery of the opening of the hollow hole 135.

The hollow hole 135 is typically applied to surgical tools, but in the present disclosure, the hollow hole 135 is regarded as an optional component together with the centering dimple 134.

The navigation marker unit 120 may include the marker fixing plate 124 having a plurality of branches, which are four in this embodiment, and the marker balls 123 provided at ends of the respective branches of the marker fixing plate 124, and the navigation marker unit 120 may be fixedly or detachably coupled to the stationary body 111 of the adapter 110 by a marker unit fixture 122 of post type.

FIG. 5 is a partial enlarged view of a head portion of the tool shaft 133, in which (A) is a front view of a head portion of the tool shaft, and (B) is a sectional view thereof.

As illustrated in (A) and (B) of FIG. 5, the coupling head 132 is provided at the head portion of the tool shaft 133, and the centering dimple 134 in the form of a recess or pocket is formed on the top surface of the coupling head. Below the coupling head 132, the locking groove 133g may be formed along an outer circumferential surface of the tool shaft 133, and below the locking groove 133g, the rotational support surface 133rg may be defined as a region where the alignment balls 118ab (see FIG. 6), described later, slide or roll. Meanwhile, as shown (B) of FIG. 5, the hollow hole 135 penetrating the tool shaft 133 in the longitudinal direction may be formed at the bottom of the centering dimple 134, and thus, the centering dimple 134 is arranged to surround the periphery of the opening of the hollow hole 135.

The centering dimple 134 and the hollow hole 135 may be regarded as optional components, and in some embodiments of the present disclosure, the hollow hole 135 may be formed to penetrate the tool shaft 133 in the longitudinal direction and the centering dimple 134 may be formed to surround the periphery of the opening of the hollow hole 135, and in other embodiments, only the centering dimple 134 may be formed without the hollow hole 135.

FIG. 6 is a schematic exploded perspective view of the adapter 110 according to an embodiment of the present disclosure.

Referring to FIG. 6, most of the components of the adapter 110 according to the present disclosure, more particular, the rotational body 114, the stationary body 111 accommodating the rotational body, and components related to the operation of the rotational body 114, are arranged on the longitudinal axis (Y-Y) extending in a vertical direction.

In the description of embodiments according to the present disclosure, the term "longitudinal axis" refers to the axis (Y-Y) extending vertically as illustrated in FIGS. 4 to 6, and the terms "upward" and "upper" refer to a direction above the longitudinal axis, whereas "downward" and "lower" refer to a direction below the longitudinal axis. Most of the components belonging to the adapter 110 may be commonly concentric about the longitudinal axis, and the respective longitudinal axes of the components may be parallel to or may commonly coincide with the longitudinal axis (Y-Y) in the vertical direction.

The navigation marker unit 120 may be installed at a side portion of the stationary body 111 with the bore cavity 111bc positioned on the longitudinal axis (Y-Y).

The navigation marker unit 120 may include the marker balls 123, the marker fixing plate 124 supporting the marker balls 123, the marker unit fixture 122 of post type connecting the marker fixing plate 124 to the stationary body 111, and a fastening component 121 fixing the plate 124 on the marker unit fixture 122.

A holding button 112 may be slidably disposed through an upper portion of the stationary body 111 in a direction orthogonal to the bore cavity 111bc, and a locking tube 115 is coupled below the holding button.

A top end 115te of the locking tube 115 may be fixed to a tubular bottom end 111be of the stationary body 111 by screw coupling or welding, and the rotational body 114 may be positioned within the bore cavity 111bc. The locking tube 115 may be regarded as a part of the stationary body that is integrally coupled with the stationary body.

The holding button 112 may allow or restrict movement in the longitudinal direction while allowing rotation of the rotational body 114. A locking groove 114g of annular shape, with which an inner edge of a locking hole 112h of the holding button 112 is engaged, may be formed just below a top end of the rotational body 114.

The locking hole 112h of the holding button 112 is larger in diameter than the rotational body 114, and when concentric with the rotational body 114, axial movement of the rotational body 114 is allowed, whereas when eccentric, the inner edge of the locking hole 112h is caught by the locking groove 114g of the rotational body 114, thereby restricting the axial movement.

The holding button 112 may be normally eccentric relative to the rotational body 114 by a bias spring 112sp, and when an external force is applied to the holding button 112 for insertion or removal of the surgical tool, the holding button becomes concentric with the rotational body 114, so that axial movement of the rotational body becomes possible and coupling or uncoupling of the tool shaft is enabled at that time.

The holding button 112 is limited in sliding distance by a sliding guide 112lb of a bolt type installed in a guide slot 112s of a preset length formed in the holding button 112.

The rotational body 114 is installed within stationary body 111 and the bore cavity 111bc defined by the locking tube 115 that may be considered a part of the stationary body 111, and a centering plunger 116 and a compression spring 116sp that elastically biases the centering plunger are provided within the rotational body 114.

The compression spring 116sp may elastically biase the centering plunger 116, which extends through a central axis of the rotational body 114 and has a head exposed at a bottom (or ceiling) of a socket portion 114sk. This plunger is to center the coupling head 132 within the socket portion 114sk by entering the hollow hole 135, the centering dimple 134, or the recess formed or provided on the top surface of the coupling head of the tool shaft.

The rotational body 114, which houses the centering plunger 116 as described above, is elastically biased upward along the longitudinal axis by the compression spring 115sp installed to surround the rotational body.

To provide the elastic biasing force, a flange 114f is formed on the rotational body 114 to abut an upper end of the compression spring 115sp, and a step portion 115st (see

FIGS. 6 and 7) to abut a lower end of the compression spring 115sp is formed approximately in a middle portion of the locking tube 115.

A locking cam 115cm of annular shape (see FIGS. 6 to 8) is formed in a lower inner portion of the locking tube 115 as a cam member for actuating the locking ball 118lb serving as an actuator, and a locking ball actuation hole 114hl in which the locking ball 118lb is positioned is formed in the rotational body 114 facing the locking cam 115cm. The locking ball actuation hole 114hl is a region where the locking ball 118lb is moved in a radial direction with respect to the longitudinal axis by the locking cam 115cm.

The locking ball 118lb is configured to press one side surface of the coupling head 132 of the surgical tool 130 with a certain force, or to be positioned in the annular locking groove 133g formed below the coupling head 132, thereby fixing the coupling head 132 in the socket portion 114sk formed at the bottom end of the rotational body 114.

The cam nut 118cn, which is a type of cam member and one component of the shaft aligner 118, is coupled to the lower end of the locking tube 115. For this coupling, the threaded portion 115th is formed on the lower end of the locking tube 115, and the threaded portion 118th is correspondingly formed on the inner circumferential surface of the cam nut 118cn. Meanwhile, an end cap 118ec is inserted and coupled to an open end of the rotational body 114 below the cam nut 118cn.

Accordingly, when the cam nut 118cn rotates relative to the locking tube 115, the cam nut 118cn moves upward toward or downward away from the locking tube 115 depending on the direction of rotation, and simultaneously, the position of the rotational body also changes.

An alignment cam 118cm of annular shape obliquely formed with respect to the longitudinal axis of the rotational body 114 may be formed on the inner circumferential surface of the cam nut 118cn, and alignment ball actuation holes 114ha for actuating alignment balls may be formed near the end of the rotational body 114, that is, near an opening of the socket portion 114sk, in correspondence with the alignment cam.

The alignment ball actuation holes 114ha may be formed in two, three, or more in arbitrary or constant angular intervals to support the tool shaft 133 coupled to the socket portion 114sk coaxially with respect to the rotational body 114.

A position of the cam nut 118cn may be adjusted according to a size of a shaft of a surgical tool having a specific standard, and a diameter or radius of a sliding or rolling support circle formed by the alignment balls 118ab that define the rotational support surface 133rg may be temporarily fixed, which may be determined by a positioner such as a set screw 118ss.

Meanwhile, the cover 113 for protecting the stationary body 111, the locking tube 115 coupled to the bottom end of the stationary body 111, and the cam nut 118cn may be installed below the stationary body 111. The cover 113 may be detachably fixed to the stationary body 111 by a stopper 117 of a push-button type that is elastically biased by a spring 117sp.

FIG. 7 is a schematic sectional view of the adapter 110 illustrated in FIG. 6 in an assembled state.

Referring to FIG. 7, the adapter 110 may include the stationary body 111, to the side portion of which the navigation marker unit 120 described above is fixed by the marker unit fixture 122 of post type, and the locking tube 115 fixed to the bottom end of the stationary body 111 to form the bore cavity. The locking tube 115 may be regarded as a part of the stationary body.

The tubular bottom end 111be of the stationary body 111 and the top end 115te of the locking tube 115 are fixed to each other by screw coupling or welding, and the rotational body 114 is positioned within the bore cavity 111bc.

In the bore cavity 111bc defined by the stationary body 111 and the locking tube 115, the rotational body 114 may be installed to be rotatable and reciprocally movable in the longitudinal direction and may be elastically biased upward by the compression spring 115sp disposed around the rotational body.

To provide the elastic biasing force, the flange 114f that abuts the upper end of the compression spring 115sp may be formed on the rotational body 114, and the step portion 115st that abuts the lower end of the compression spring 115sp may be formed approximately in the middle portion of the locking tube 115.

On an inner surface of the locking tube 115, the locking cam 115cm of annular shape, which is a cam member for actuating the locking ball 118lb serving as an actuator, is formed near the step portion 115st, and, in the figure, below the step portion. The locking ball actuation hole 114hl, in which the locking ball 118lb is located, is formed in the rotational body 114 facing the locking cam 115cm. The locking ball actuation hole 114hl is a region where the locking ball 118lb, which is interfered with by the locking cam 115cm, moves.

The locking ball 118lb presses a side surface of the coupling head 132 of the surgical tool 130, or fixes the coupling head 132 to the socket portion 114sk while positioned in the annular locking groove 133g located below the coupling head 132. A surface of the locking cam 115cm, which interferes with the locking ball 118lb, may be inclined with respect to the longitudinal axis of the rotational body 114, and, depending on a position of the locking cam 115cm, may push the locking ball 118lb toward a center of the rotational body 114 or release the pressing action.

In the bore cavity 111bc, when the rotational body 114, which is elastically biased toward the stationary body 111 by the compression spring 115sp with respect to the locking tube 115, is pushed downward in one direction (downward in the figure) by the shank 114sh, the rotational body 114 overcomes the elastic force of the compression spring 115sp and moves downward in the bore cavity 111bc.

According to such an operation, as the rotational body 114 moves relative to the locking tube 115 or the stationary body 111, the locking ball 118lb, which is positioned in the locking groove 133g located under the coupling head 132 due to the locking cam 115cm obliquely formed on the inner surface of the locking tube 115, escapes from the locking groove 133g, so that insertion and removal of the coupling head 132 of the surgical tool 130 into and from the socket portion 114sk provided at one side (lower side in the figure) of the rotational body 114 become possible. In this operation, the alignment balls 118ab, which will be described later and which perform a sliding or rolling motion with respect to the rotational support surface 133rg of the coupling head 132, are also spaced apart from the rotational support surface.

The cam nut 118cn, which is a type of cam member and one element of the shaft aligner 118, is coupled to the lower end of the locking tube 115, with the threaded portion 115th formed on the lower end of the locking tube 115 and the corresponding threaded portion 118th formed on the inner circumferential surface of the cam nut 118cn for this coupling.

Accordingly, when the cam nut 118cn rotates relative to the locking tube 115, the cam nut 118cn moves upward toward or downward away from the locking tube 115 depending on directions of rotation, and simultaneously, a position of the rotational body also changes.

The end cap 118ec is fitted and coupled to the opening of the socket portion 114sk at the lower end of the rotational body 114.

The cam nut 118cn includes, on an inner surface of the cam nut 118cn, an annular alignment cam 118cm that is obliquely formed with respect to the longitudinal axis of the rotational body 114 to align the alignment balls 118ab so that the alignment balls are slidable or rollable with respect to the rotational support surface 133rg of the tool shaft 133, and, in correspondence with the alignment cam 118cm, the alignment ball actuation holes 114ha for operating the alignment balls are formed near a lower end of the rotational body 114, that is, near the opening of the socket portion 114sk.

According to the above structure, rotation of the cam nut 118cn as a cam member relative to the locking tube 115 causes a contact region of the alignment cam 118cm with respect to the alignment balls 118ab located in the alignment ball actuation holes 114ha of the locking tube 115 to change along the inclined surface of the locking cam 115cm, thereby increasing or decreasing a diameter of a circle circumscribed by the alignment balls 118ab.

The actuation holes 114ha may be formed in two, three, or more in arbitrary or constant angular intervals to support the tool shaft 133 coupled to the socket portion 114sk coaxially with respect to the rotational body 114.

A position of the cam nut 118cn may be adjusted according to a size of a shaft of a surgical tool having a specific standard, and a diameter or radius of a sliding or rolling support circle formed by the alignment balls 118ab that define the rotational support surface 133rg may be temporarily fixed, which may be determined by the positioner such as the set screw 118ss.

That is, by adjusting a coupling position of the cam nut 118cn with respect to the locking tube 115, a spacing between the alignment balls 118ab is determined by the annular cam surface of the cam nut 118cn, specifically, a diameter or radius of a sliding or rolling support circle defined by the alignment balls 118ab and circumscribed around them is determined, and in this state, a coupling or fastening position of the cam nut 118cn to the locking tube 115 is fixed by using the set screw 118ss.

According to another embodiment, the cam nut 118cn may be modified into another form having a function as a cam member. For example, instead of a linear transfer structure using screw coupling, it is also possible to determine a position of the alignment cam 118cm relative to the alignment ball actuation holes 114ha of the rotational body 114 by a simple surface contact-type press fit without screws, and then fix this position with the set screw 118ss.

The centering plunger 116 elastically biased by the compression spring 116sp may be installed at the bottom (an upper ceiling in the figure) of the socket portion 114sk. The centering plunger 116 passes through a longitudinal axis of the rotational body 114 and has the head portion exposed at the bottom (or ceiling) of the socket portion.

This plunger is to center the coupling head 132 within the socket portion 114sk by entering the hollow hole 135, the concave centering dimple 134 formed or provided on the top surface of the coupling head of the tool shaft. According to this centering structure, even when a size of the coupling head is slightly smaller than that of the socket portion, the coupling head may be centrally aligned within the socket portion.

Meanwhile, the upper portion of the stationary body 111 may be provided with the holding button 112 of a slider type that is slidable in a direction orthogonal to the longitudinal axis of the rotational body 114 to allow rotation of the rotational body 114 while permitting or restricting axial movement, and near the top end of the rotational body 114, the locking groove 114g of annular shape may be formed, in correspondence with the holding button 112, in which the inner edge of the locking hole 112h of the holding button 112 is engaged.

The locking hole 112h of the holding button 112 may be larger in diameter than the rotational body 114, and when concentric with the rotational body 114, axial movement of the rotational body 114 is permitted, but when eccentric, the inner edge of the locking hole 112h is caught by the locking groove 114g of the rotational body 114, thereby restricting the axial movement.

The holding button 112 is normally eccentric with respect to the rotational body 114 by the bias spring 112sp, and when an external force is applied to the holding button 112 for insertion or removal of a surgical tool, the button becomes concentric with the rotational body 114, thereby allowing axial movement of the rotational body 114, and enabling coupling or decoupling of the tool shaft.

The holding button 112 is limited in sliding distance by the sliding guide 112lb of a bolt type installed in the guide slot 112s of a preset length formed in the holding button 112.

Meanwhile, the stationary body 111, the locking tube 115 coupled to the lower end thereof, and the cam nut 118cn may be protected by the cover 113, which may be detachably coupled to the stationary body 111 by the stopper 117 of a push-button type or plunger type that is elastically biased by the spring 117sp.

FIG. 7 illustrates a state in which the tool shaft 133 is not coupled to the socket portion 114sk for better understanding of the socket portion 114sk, but shows a state in which the locking ball 118lb is positioned within the locking groove 133g beneath the coupling head 132 of the tool shaft 133 and the alignment balls 118ab may be in contact with the rotational support surface of the tool shaft 133 to allow sliding or rolling movement, which may be understood as such.

In another embodiment, locking means including the locking ball 118lb and the locking cam 115cm, which function to temporarily hold the coupling head 132 to the rotational body 114, may be omitted, and only the shaft aligner for the rotational support surface of the tool shaft may be applied.

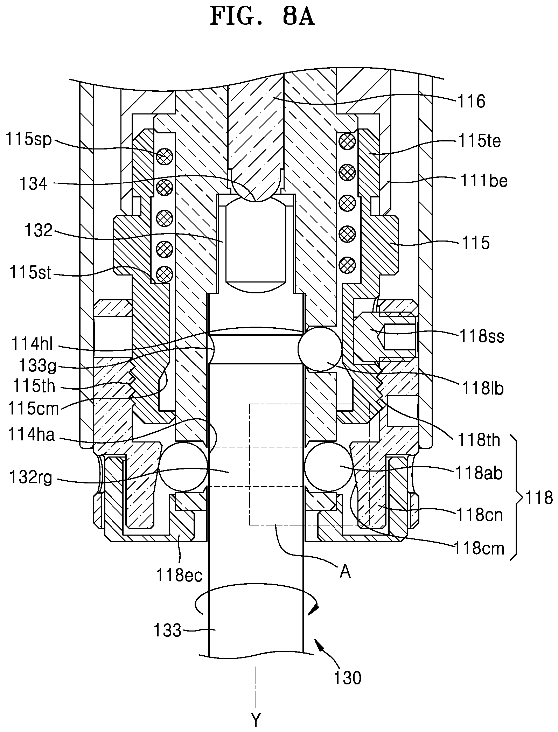

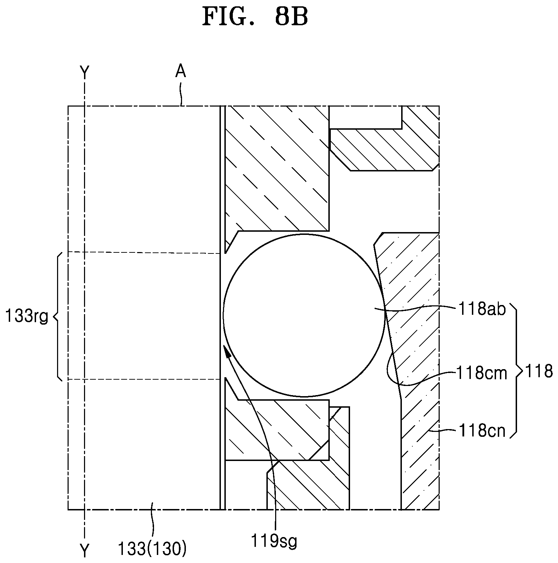

FIG. 8A is a partial view showing a state in which a tool shaft is rotatably supported in the adapter illustrated in FIG. 5, and FIG. 8B is a partially enlarged view showing a state in which alignment balls support a support surface of the tool shaft to allow rolling or sliding motion.

As illustrated in FIGS. 8A and 8B, the alignment balls 118ab are positioned adjacent to the rotational support surface 133rg of the tool shaft 133 with a rolling or sliding gap 119sg therebetween. In this state, the tool shaft 133 is rotatably supported within the socket portion 114sk by the alignment balls 118ab while maintaining coaxiality with the rotational body 114. As exemplarily shown in FIG. 7A, a diameter of the shaft 133 is slightly smaller than an inner diameter of the socket portion 114sk, and thus a clearance exists between them. In this state, the centering plunger 116 strongly presses the centering dimple 134 of the coupling head 132 of the tool shaft 133 to center the coupling head 132 within the socket portion 114sk, and the alignment balls 118ab, which face or contact the rotational support surface 133rg of the tool shaft 133 with the sliding gap 119sg therebetween, support the rotational support surface 133rg to allow sliding or rolling, thereby suppressing tilting of the tool shaft 133.

A position of the alignment balls 118ab is adjusted by the shaft aligner 118 having the cam member axially movably coupled to the locking tube 115, and, in an embodiment, by the cam nut 118cn having the alignment cam 118cm in contact with the alignment balls 118ab, and accordingly, a spacing or contact pressure of the alignment balls 118ab with respect to the rotational support surface 133rg is controlled.

In this embodiment, the cam nut 118cn screw-coupled to the locking tube 115 is employed to adjust the position of the alignment balls 118ab, specifically a radius of the array of the alignment balls.

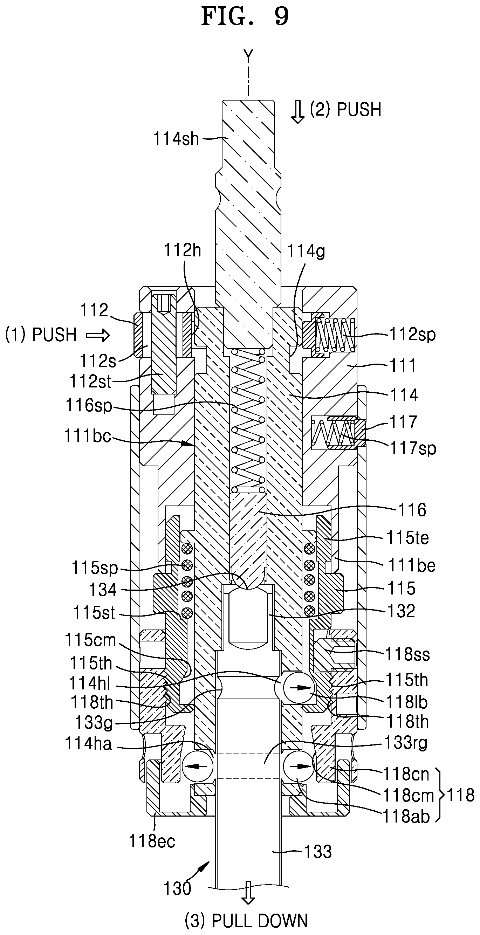

FIG. 9 illustrates a state of the adapter 110 according to the present disclosure in which a surgical tool can be replaced, that is, a released state in which the locking ball and the alignment ball are spaced apart from the coupling head 132 of the tool shaft and the rotational support surface 133rg.

Referring to FIG. 9, to remove the surgical tool 130 coupled to the socket portion 114sk, the holding button 112 may be first pressed to release axial locking with respect to the rotational body 114, and then the shank 114sh of the rotational body 114 may be pressed to disengage the locking ball 118lb and the alignment balls 118ab provided at the lower portion of the rotational body 114 from the cam surfaces of the respective cams 115cm and 118cm. As a result, the locking ball 118lband the alignment balls 118ab move from a narrow-diameter annular cam surface to a wide-diameter region (i.e., downward in the drawing), so that the coupling head 132 and the rotational support surface 133rg of the surgical tool 130 may be easily separated from the socket portion 114sk. In this state, because the locking ball 118lb and the alignment balls 118ab have moved from a narrow-diameter portion of the annular cam surface to a wide-diameter portion, the tool or another tool may be firstly inserted, and then, by removing the pressing force applied to the shank, locking of the coupling head 132 by the locking ball 118lb may be achieved.

In mounting a new surgical tool on the adapter 110, when adjustment of spacing of the alignment balls 118ab installed around the socket portion 114sk is required, the cam nut 118cn described above may be adjusted to allow insertion of the new tool.

After mounting a new surgical tool in the socket portion 114sk, when unstable radial runout, such as radial shaking of the surgical tool, still remains, a spacing of the alignment balls 118ab may be adjusted while the tool remains mounted, and when the shaking is reduced below a certain level while allowing rotation of the shaft through such adjustment, the spacing of the alignment balls 118ab may be fixed as a positioning state. As one method for this, the cam nut 118cn may be tightened so that the alignment balls 118ab are fully brought into contact with the rotational support surface 133rg of the tool shaft 133, and then slightly loosened in a direction opposite to the tightening direction, so that the pressing force is reduced while maintaining a contact state that allows sliding or rolling movement with the alignment balls 118ab that have pressed the rotational support surface 133rg being retracted.

As defined above and shown in various forms in FIGS. 4 to 6, the driver according to the present disclosure may accommodate the navigation marker unit without modifying a size of the driver shaft. Due to fine processing errors caused by variations in manufacturers or production lots, the outer diameter of screwdrivers may slightly vary, but the driver according to the present disclosure overcomes such variations and is compatible with most heterogeneous driver shafts.

In the case of a driver to which a navigation marker unit is attached, the greater the clearance between shafts, the lower the positioning accuracy of the navigation becomes, and conversely, the smaller the clearance, the higher the positioning accuracy becomes, and according to the present disclosure, this clearance may be eliminated to provide rotational support for the shaft in a firm and stable state.

A coupling head (mounting portion of the shaft) to be mounted to a handle or a marker body is provided at an end of the shaft, and such coupling heads are generally classified into 1/4' Square, Hudson, and Jacobs chuck types, and in this embodiment, the specification of the 1/4' Square chuck mounting portion is used as a reference.

In the surgical driver according to the present disclosure, the navigation marker may be mounted on a portion where a handle of a screwdriver is mounted, that is, a 1/4' Square chuck. The handle may be mounted on the 1/4' Square chuck provided on the upper portion of the stationary body in the form of the adapter to which the navigation marker is attached.

A mechanism for holding a navigation marker and a shaft with an appropriate clearance may be implemented by the shaft aligner that includes a plurality of actuators, for example, three actuators such as the alignment balls arranged at arbitrary angular intervals, the radius of the array of which may vary depending on the cam nut having the inclined cam surface, thereby enabling stable support of the shaft positioned between the actuators and allowing sliding or rolling movement.

The shaft aligner may have a structure similar to a collet; however, unlike typical collet structures in which a shaft is tightly fixed, the shaft aligner may allow sliding or rolling movement between a plurality of actuators of the collet, for example, ball-shaped actuators, and the shaft.

That is, the shaft aligner may adopt the basic structure of a well-known collet, but may be configured to hold the shaft to a degree sufficient to prevent transverse shaking along the shaft axis, thereby allowing the shaft to rotate relative to the stationary body by torque transmitted through the handle.

A cam, or a cam-shaped longitudinal transfer member functioning as the cam nut, may be coupled to the rotational body so as to allow threaded engagement or relative linear transfer, and may be configured to reciprocate along the longitudinal axis of the shaft by a desired distance depending on a degree and direction of rotation or advancement. In this case, the inclined cam surface of the cam nut presses or releases the inner ball actuators within the cam nut, for example, the alignment balls described above, so that the ball actuator comes into slidable contact with or moves away from the rotational support surface of the shaft.

The ball-type actuator that moves in a radial direction orthogonal to the longitudinal axis of the tool shaft by the cam surface as described above may be replaced with a rotatable rod-shaped actuator having a cylindrical, conical, or truncated conical shape rather than a spherical shape.

However, in the embodiment, the above-described alignment balls or other types of actuators in the form of balls may be applied as the actuators that allow sliding and/or rolling motion with ease. That is, in the embodiment, a rotational support structure of a chucking structure may be configured to rotatably support the shaft by actuators having various forms.

It is preferable that three actuators of such forms are arranged at equal angular intervals, although two may be applied with the aid of some support structure, or more preferably, three or more may be used.

Inside the cam nut, actuators, for example, three ball actuators, are positioned as described above, and as the cam nut rotates, the internal actuators may be interfered with by the inclined cam surface formed on the inner circumferential surface of the cam nut, and may thereby approach or move away from the surface of the shaft.

Because the set screw is provided in the cam nut, when the shaft is pressed with an appropriate pressure by the actuators through rotation of the cam nut to a degree that allows rotation, this state may be fixed by the set screw.

Meanwhile, on the inner circumferential surface of the cam nut, a plurality of tap holes in which positioning components such as set screws may be installed, as illustrated in FIG. 6, are formed for fixing an inner diameter, for example, twelve tap holes are formed at 30-degree intervals, and when the cam nut rotates and moves by one tap hole, the inner diameter defined by three balls may be adjusted by ±0.03 mm. Accordingly, after the 1/4' square chuck of the screwdriver shaft is mounted to the stationary body to which the navigation marker is attached, components of the inclined portion may be rotated to adjust an appropriate clearance, and once the clearance is determined, the inner diameter may be fixed by securing the tap hole at a specific rotational position to the stationary body or to a locking tube elastically supported on the stationary body by using the set screw, so that the shaft is rotatably supported by the actuators. By fixing a position of the tap hole with the set screw as described above, the inner diameter is appropriately defined, enabling the shaft to rotate stably without rotational wobble.

For navigation accuracy, not only clearance between components but also securing concentricity of the driver shaft with respect to the stationary body to which the navigation marker is mounted is important.

In the present disclosure, to ensure concentricity inside the rotor, a centering plunger corresponding to a hollow hole or a centering dimple formed in a head of the shaft is applied to firmly maintain coaxiality of the shaft with respect to the rotational body.

While various embodiments of the present invention have been described in detail above, it will be apparent to those of ordinary skill in the art that the present invention may be modified and implemented in various ways without departing from the spirit and scope of the invention as defined in the appended claims. Therefore, future modifications of the embodiments of the present invention will not depart from the technical scope of the present invention.

## Claims

1. A surgical tool adapter for compatibly mounting surgical tools, the surgical tool adapter comprising:
a rotational body having a socket portion to which a coupling head provided on a tool shaft of a surgical tool is detachably coupled;
a stationary body having a bore cavity in which the rotational body is rotatably mounted; and
a shaft aligner configured to align the tool shaft coaxially with a longitudinal axis of the rotational body while allowing rotation of the rotational body and the coupled tool shaft within the bore cavity of the stationary body to thereby control radial runout of the tool shaft.

2. The surgical tool adapter of claim 1, wherein the shaft aligner comprises:
a plurality of actuators movably disposed to contact and separate from a rotational support surface defined on an outer circumferential surface of the tool shaft to support rotation of the shaft; and
a positioner configured to align the plurality of actuators with the rotational support surface of the tool shaft.

3. The surgical tool adapter of claim 2, wherein the plurality of actuators are disposed around the tool shaft to be movable in a radial direction with respect to a longitudinal axis of the tool shaft so as to approach or move away from the rotational support surface of the shaft.

4. The surgical tool adapter of claim 3, wherein the plurality of actuators are rotatable members configured to perform a sliding or rolling movement with respect to the rotational support surface of the shaft.

5. The surgical tool adapter of claim 4, wherein the plurality of actuators each have a shape of one of a ball, a cylinder, a cone, or a truncated cone having a surface that partially contacts the rotational support surface of the shaft.

6. The surgical tool adapter of claim 4, wherein a plurality of actuation holes in which the plurality of actuators are radially movable are formed at arbitrary angular intervals around an opening of the socket portion into which the shaft is inserted;
the positioner includes a cam member configured to press or release the plurality of actuators located in the plurality of actuation holes to bring the actuators into contact with and separate the actuators from the rotational support surface of the shaft in the socket portion; and
the cam member includes an inclined cam surface configured to push the plurality of actuators toward a center of the shaft and move the actuators away from the center.

7. The surgical tool adapter of claim 6, wherein the cam member is provided to be movable in a longitudinal direction of the rotational body to push the plurality of actuators toward or move the actuators away from the center of the shaft according to a position of the cam member in the longitudinal direction.

8. The surgical tool adapter of claim 6, further comprising a locking tube integrally coupled with the stationary body and forming a portion of a bore cavity accommodating the rotational body,
wherein the cam member is coupled to an end of the locking tube to be movable in a longitudinal direction and configured to move the plurality of actuators toward or away from the rotational support surface depending on a longitudinal position of the cam member relative to the locking tube or the stationary body.

9. The surgical tool adapter of claim 8, wherein the stationary body, the cam member, and the locking tube therebetween are arranged substantially concentrically on a single axis;
the bore cavity is configured to accommodate the rotational body to be reciprocally movable along the longitudinal axis, and the plurality of actuators interfering with the rotational body are configured to be disengaged from the cam member as the rotational body moves in the longitudinal direction to release locking of the shaft in the socket portion, a compression spring is positioned inside the locking tube to elastically bias the rotational body, provide a reaction force against a movement of the rotational body toward the cam member, and restore a position of the rotational body; and
the cam member is threadably coupled to one longitudinal end of the stationary body or the locking tube and is configured such that a position of the cam member with respect to the plurality of actuators is moved in the longitudinal direction according to a rotation degree of the cam member.

10. The surgical tool adapter of claim 2, wherein the positioner comprises:
a cam member including a cam having an inclined cam surface surrounding an array of the plurality of actuators;
a cam holder configured to fix a position of the cam member according to a determination by the positioner; and
wherein the cam surface is inclined with respect to the longitudinal axis of the shaft so as to push the plurality of actuators toward or away from the rotational support surface of the shaft to thereby form the surgical tool adapter.

11. The surgical tool adapter of claim 2, wherein the positioner comprises:
a linear feeder configured to be reciprocally movable along the longitudinal axis with respect to the rotational body; and
a cam holder including a set screw for fixing a cam member including a cam having an inclined cam surface to thereby form the surgical tool adapter.

12. The surgical tool adapter of claim 1, wherein the socket portion is located
on a central axis of the rotational body; and
a plunger configured to press a top surface of the coupling head is provided on a bottom of the socket portion corresponding to the top surface of the coupling head to thereby form the surgical tool adapter.

13. The surgical tool adapter of claim 12, wherein the plunger is configured to be elastically biased by a spring to elastically press a top surface of a polygonal head.

14. The surgical tool adapter of claim 9, wherein a hollow hole, a centering dimple, a centering pocket, or a centering recess into which a portion of a plunger enters is formed in a top surface of the coupling head to thereby form the surgical tool adapter.

15. A surgical tool driver comprising:
a surgical tool having a tool shaft provided with a tool tip at a front end and a coupling head at a rear end;
a surgical tool adapter according to any one of claims 1 to 14 and including a rotational body to which the surgical tool is detachably coupled; and
a surgical tool driver including a handle coaxially connected to a rotating rod provided in the rotational body.

16. The surgical tool driver of claim 15, wherein a shank is coaxially coupled to the rotational body, and a handle including a ratchet capable of controlling a rotational direction is detachably coupled to the shank.

17. The surgical tool driver of claim 15, wherein
a navigation marker for surgical navigation is attached to the stationary body.

18. The surgical tool driver of claim 17, wherein the navigation marker comprises:
a plurality of optically detectable markers;
a marker plate to which the plurality of markers are fixed; and
a support configured to connect the marker plate to the stationary body.

19. A method of compatibly mounting surgical tools on a surgical tool driver, the method comprising:
preparing a surgical tool having a tool shaft provided with a tool tip at a front end and a coupling head at a rear end;
installing a rotational body rotatably within a bore cavity formed in a stationary body;
coupling the coupling head of the tool shaft to a socket portion provided in the rotational body; and
supporting the tool shaft coupled to the rotational body to be rotatable with respect to the stationary body, allowing a rotation of the rotational body and the tool shaft within the bore cavity of the stationary body, and aligning the tool shaft with respect to the rotational body by controlling a radial runout of the tool shaft by using a driver shaft aligner.

20. The method of claim 19, further comprising:
actuating a cam member having a cam surface surrounding an array of a plurality of actuators to allow the plurality of actuators to rotatably support a rotational support surface of the tool shaft using the cam surface.

21. The method of claim 20, further comprising:
moving the cam member in one or another direction along a longitudinal axis of the tool shaft, and pressing or releasing the actuators against the rotational support surface of the tool shaft using the cam surface formed obliquely with respect to the longitudinal axis of the tool shaft.

## Amended claims

### Amended claims under Art. 19.1 PCT

1. A surgical tool adapter compatible with surgical tools having tool shafts of different specifications, the surgical tool adapter comprising:
a rotational body having a socket portion to which coupling heads provided on tool shafts of the surgical tools are detachably coupled;
a stationary body having a bore cavity in which the rotational body is rotatably mounted; and
a shaft aligner configured to align the tool shafts coaxially with a longitudinal axis of the rotational body while allowing rotation of the rotational body and a tool shaft positioned within the bore cavity of the stationary body, the shaft aligner comprising movable actuators configured to contact an outer circumferential surface of the tool shaft to rotatably support the tool shaft,
wherein positions of the actuators are determined according to a diameter of the tool shaft to control radial runout of the tool shaft within a certain range.

2. The surgical tool adapter of claim 1, wherein the shaft aligner comprises:
a plurality of actuators movably disposed to contact and separate from a rotational support surface defined on the outer circumferential surface of the tool shaft to support rotation of the tool shaft; and
a positioner configured to align the plurality of actuators with the rotational support surface of the tool shaft.

3. The surgical tool adapter of claim 2, wherein the plurality of actuators are disposed around the tool shaft to be movable in a radial direction with respect to a longitudinal axis of the tool shaft so as to approach or move away from the rotational support surface of the shaft.

4. The surgical tool adapter of claim 3, wherein the plurality of actuators are rotatable members configured to perform a sliding or rolling movement with respect to the rotational support surface of the shaft.

5. The surgical tool adapter of claim 4, wherein the plurality of actuators each have a shape of one of a ball, a cylinder, a cone, or a truncated cone having a surface that partially contacts the rotational support surface of the shaft.

6. The surgical tool adapter of claim 4, wherein a plurality of actuation holes in which the plurality of actuators are radially movable are formed at arbitrary angular intervals around an opening of the socket portion into which the shaft is inserted;
the positioner includes a cam member configured to press or release the plurality of actuators located in the plurality of actuation holes to bring the actuators into contact with and separate the actuators from the rotational support surface of the shaft in the socket portion; and
the cam member includes an inclined cam surface configured to push the plurality of actuators toward a center of the shaft and move the actuators away from the center.

7. The surgical tool adapter of claim 6, wherein the cam member is provided to be movable in a longitudinal direction of the rotational body to push the plurality of actuators toward or move the actuators away from the center of the shaft according to a position of the cam member in the longitudinal direction.

8. The surgical tool adapter of claim 6,
further comprising a locking tube integrally coupled with the stationary body and forming a portion of a bore cavity accommodating the rotational body,
wherein the cam member is coupled to an end of the locking tube to be movable in a longitudinal direction and configured to move the plurality of actuators toward or away from the rotational support surface depending on a longitudinal position of the cam member relative to the locking tube or the stationary body.

9. The surgical tool adapter of claim 8, wherein the stationary body, the cam member, and the locking tube therebetween are arranged substantially concentrically on a single axis;
the bore cavity is configured to accommodate the rotational body to be reciprocally movable along the longitudinal axis, and the plurality of actuators interfering with the rotational body are configured to be disengaged from the cam member as the rotational body moves in the longitudinal direction to release locking of the tool shaft in the socket portion,
a compression spring is positioned inside the locking tube to elastically bias the rotational body, provide a reaction force against a movement of the rotational body toward the cam member, and restore a position of the rotational body; and
the cam member is threadably coupled to one longitudinal end of the stationary body or the locking tube and is configured such that a position of the cam member with respect to the plurality of actuators is moved in the longitudinal direction according to a rotation degree of the cam member.

10. The surgical tool adapter of claim 2, wherein the positioner comprises:
a cam member including a cam having an inclined cam surface surrounding an array of the plurality of actuators;
a cam holder configured to fix a position of the cam member according to a determination by the positioner; and
wherein the cam surface is inclined with respect to the longitudinal axis of the shaft so as to push the plurality of actuators toward or away from the rotational support surface of the shaft.

11. The surgical tool adapter of claim 2, wherein the positioner comprises:
a linear feeder configured to be reciprocally movable along the longitudinal axis with respect to the rotational body; and
a cam holder including a set screw for fixing a cam member including a cam having an inclined cam surface.

12. The surgical tool adapter of claim 1, wherein the socket portion is located on a central axis of the rotational body; and
a plunger configured to press a top surface of the coupling head is provided on a bottom of the socket portion corresponding to the top surface of the coupling head to form the surgical tool adapter.

13. The surgical tool adapter of claim 12, wherein the plunger is configured to be elastically biased by a spring to elastically press a top surface of a coupling head.

14. The surgical tool adapter of claim 9, wherein a plunger configured to press a top surface of the coupling head is provided on a bottom of the socket portion corresponding to the top surface of the coupling head, and a hollow hole, a centering dimple, a centering pocket, or a centering recess into which a portion of a plunger enters is formed in a top surface of the coupling head.

15. A surgical tool driver comprising:
a surgical tool having a tool shaft provided with a tool tip at a front end and a coupling head at a rear end;
a surgical tool adapter compatible with surgical tools having tool shafts of different specifications; and
a handle coaxially connected to a rotational rod provided in the rotational body;
wherein the surgical tool adapter comprises:
a rotational body having a socket portion to which coupling heads provided on tool shafts of the surgical tools are detachably coupled;
a stationary body having a bore cavity in which the rotational body is rotatably mounted; and
a shaft aligner configured to align the tool shafts coaxially with a longitudinal axis of the rotational body while allowing rotation of the rotational body and a tool shaft positioned within the bore cavity of the stationary body, the shaft aligner comprising movable actuators configured to contact an outer circumferential surface of the tool shaft to rotatably support the tool shaft,
wherein positions of the actuators are determined according to a diameter of the tool shaft to control radial runout of the tool shaft within a certain range.

16. The surgical tool driver of claim 15, wherein a shank is coaxially coupled to the rotational body, and a handle including a ratchet capable of controlling a rotational direction is detachably coupled to the shank.

17. The surgical tool driver of claim 15, wherein a navigation marker for surgical navigation is attached to the stationary body.

18. The surgical tool driver of claim 17, wherein the navigation marker comprises:
a plurality of optically detectable markers;
a marker plate to which the plurality of markers are fixed; and
a support configured to connect the marker plate to the stationary body.

19. The surgical tool driver of claim 15, wherein the shaft aligner comprises:
a plurality of actuators movably disposed to contact and separate from a rotational support surface defined on the outer circumferential surface of the tool shaft to support rotation of the tool shaft; and
a positioner configured to align the plurality of actuators with the rotational support surface of the tool shaft.

20. The surgical tool driver of claim 19, further comprising:
the plurality of actuators disposed around the tool shaft to be movable in a radial direction with respect to a longitudinal axis of the tool shaft so as to approach or move away from the rotational support surface of the shaft.
